# FASCICULE DE BREVET EUROPEEN

(11) **EP 2 115 488 B1**
(45) Date de publication et mention de la délivrance du brevet: **26.08.2015**
(21) Numéro de dépôt: 08762013.4
(22) Date de dépôt: 30.01.2008
(51) Int. Cl.: G01T 1/161, G01T 1/20

(54) **DISPOSITIF DE DOSIMETRIE IN VIVO**
IN-VIVO-DOSIMETRIEVORRICHTUNG
IN VIVO DOSIMETRY DEVICE

(30) Priorité: 30.01.2007 FR 0752962
(43) Date de publication de la demande: 11.11.2009
(73) Titulaire: Université Claude Bernard Lyon I, 69622 Villeurbanne Cedex (FR); Centre Hospitalier Universitaire de Grenoble, 38043 Grenoble Cedex 9 (FR); Ecole Superieure De Chimie Physique Electronique De Lyon, 69616 Villeurbanne Cedex (FR); Universite Joseph Fourier Grenoble I, 38041 Gieres Cédex 98 (FR)
(72) Inventeur: LU, Guo-Neng, F-69100 Villeurbanne (FR); PITTET, Patrick, F-69270 Fontaines Saint Martin (FR); GALVAN, Jean-Marc, F-69100 Villeurbanne (FR)
(74) Mandataire: Thibault, Jean-Marc
(86) Numéro de dépôt international: PCT/FR2008/050153
(87) Numéro de publication internationale: WO 2008/125759

(56) Documents cités:
- WO-A-01/49162
- FR-A- 2 822 239
- US-A- 4 931 646
- US-A1- 2004 069 950
- US-B1- 6 643 538
- FERUGLIO S ET AL: "Exact Noise Analysis of a CMOS BDJ APS" CIRCUITS AND SYSTEMS, 2005. ISCAS 2005. IEEE INTERNATIONAL SYMPOSIUM ON KOBE, JAPAN 23-26 MAY 2005, PISCATAWAY, NJ, USA,IEEE, 23 mai 2005 (2005-05-23), pages 2337-2340, XP010816136 ISBN: 0-7803-8834-8
- DAGHIGHIAN F ET AL: "INTRAOPERATIVE BETA PROBE: A DEVICE FOR DETECTING TISSUE LABELED WITH POSITRON OR ELECTRON EMITTING ISOTOPES DURING SURGERY" MEDICAL PHYSICS, AIP, MELVILLE, NY, US, vol. 21, no. 1, janvier 1994 (1994-01), pages 153-157, XP000428977 ISSN: 0094-2405
- KWAK S-W ET AL: "Comparative study of CWO and ZnSe(Te) scintillation detector on the performance of X-ray imaging system" NUCLEAR INSTRUMENTS & METHODS IN PHYSICS RESEARCH, SECTION - A: ACCELERATORS, SPECTROMETERS, DETECTORS AND ASSOCIATED EQUIPMENT, ELSEVIER, AMSTERDAM, NL, vol. 537, no. 1-2, 21 janvier 2005 (2005-01-21), pages 449-453, XP004708894 ISSN: 0168-9002

## Description

La présente invention concerne le domaine technique de la dosimétrie in vivo invasif ou non invasif pour la radiothérapie externe ou le radiodiagnostic.

L'objet de l'invention vise plus précisément un dispositif de dosimétrie comportant une sonde miniaturisée permettant de mesurer une dose d'un rayonnement haute énergie.

Dans le domaine préféré de la dosimétrie in vivo en radiothérapie, il apparaît le besoin de mesurer le rayonnement haute énergie apparaissant sur les zones anatomiques irradiées volontairement et/ou sur les zones anatomiques périphériques radiosensibles. Cette mesure in vivo permet une vérification de la dose délivrée et/ou une appréciation de l'impact du rayonnement haute énergie dans l'environnement de la zone irradiée.

Dans l'état de la technique, il est connu une première catégorie de dosimètre du type semi-conducteur, tel que décrit, par exemple, dans les brevets US 5 949 075, US 5 587 199 ou dans l'article faisant un état de l'art intitulé « Electronic Dosimetry in Radiation Therapy » (Radiation Measurements, Volume 41, Supplément 1, 1 Décembre 2006, Pages S134-S153). Ce type de dosimètre comporte une cellule de détection comportant un dispositif semi-conducteur tel qu'une diode ou un transistor MOSFET permettant une conversion des photons ou particules haute énergie en électrons. L'utilisation d'une cellule de conversion de type radioélectrique nécessite la mise en oeuvre d'interconnexions électriques très pénalisantes en termes de miniaturisation et d'immunité aux perturbations électromagnétiques. Par ailleurs, ce type de dosimètre présente l'inconvénient d'être sensible à l'orientation de la cellule de détection par rapport au faisceau haute énergie.

Il est connu par ailleurs, notamment par le brevet FR 2 822 239 et par l'article faisant un état de l'art intitulé « Optically Stimulated Luminescence And Its Use In Medical Dosimetry» (Radiation Measurements, Volume 41, Supplément 1, 1 Décembre 2006, Pages S78-S99), une deuxième catégorie de dosimètres à matériaux scintillants isolants. Ce type de dosimètre comporte, en tant que cellule de détection, un matériau scintillant isolant assurant la conversion des photons ou particules haute énergie en photons ultraviolets, visibles ou infrarouges. Le signal luminescent émis par ce matériau scintillant isolant est transmis via une fibre optique, à un photodétecteur réalisant une conversion photoélectrique. Il est à noter que la conversion des photons ou particules haute énergie en photons ultraviolets, visibles ou infrarouges fait intervenir des centres de recombinaison radiatifs dans la bande interdite, ce qui conduit à un rendement de conversion relativement faible. Pour augmenter ce rendement de conversion, il est connu d'avoir recours à une stimulation thermique ou optique. Cependant, la mise en oeuvre d'une stimulation thermique ou optique complexifie la réalisation du dosimètre et nuit à la miniaturisation d'un tel dosimètre.

L'état de la technique comprend également des sondes dosimétriques à base de fibres scintillantes qui permettent une détection sans recourir à une stimulation optique ou thermique comme cela apparaît notamment dans l'article intitulé « Plastic scintillation dosimetry and its application to radiotherapy » (Radiation Measurements, Volume 41, Supplément 1, 1 Décembre 2006, Pages S124-S133). Cependant, le faible rendement de luminescence ne permet d'obtenir qu'une miniaturisation limitée de l'élément scintillant.

Dans un autre domaine de la technique notamment dans le brevet US 6,643,538, l'utilisation des matériaux semiconducteurs II-VI CdTe, CdZnTe, HgI₂ pour leurs propriétés de scintillation est décrite pour guider une sonde endoscopique chirugicale sur une tumeur radiomarquée. Cette sonde comporte un canal optique qui augmente le diamètre de la sonde, ce qui ne permet pas d'obtenir une sonde miniaturisée. Par ailleurs, les matériaux semi-conducteurs préconisés ont un numéro atomique élevé, ce qui est à même de perturber l'homogénéité de la dose d'irradiation au voisinage du matériau scintillant, ce qui limite l'intérêt de ces matériaux pour la dosimétrie in vivo in situ.

L'objet de l'invention vise donc à remédier aux inconvénients de l'art antérieur en proposant un nouveau dispositif pour la dosimétrie in vivo présentant un faible coût de revient, tout en comportant une sonde miniaturisée apte à réaliser des mesures précises.

Par ailleurs, le document WO 01/49162 décrit un dispositif d'imagerie intravasculaire conçu pour identifier et localiser des marqueurs radiopharmaceutiques dans une cavité du corps humain. Ce dispositif comporte une sonde miniaturisée comprenant un réseau de détecteurs pour détecter les marqueurs radiopharmaceutiques. Ce réseau de détecteurs comporte une pluralité de scintillateurs montés à l'extrémité distale d'une fibre optique dont l'extrémité proximale est couplée à un photodétecteur relié à une unité d'acquisition de données.

Pour atteindre un tel objectif, l'objet de l'invention concerne un dispositif pour la dosimétrie in vivo, conforme à la revendication 1.

Selon un exemple de réalisation, le matériau radioluminescent GaN est placé dans une cavité de détection montée à l'extrémité d'une fibre optique ou réalisée à l'une des extrémités d'une fibre optique pour former une sonde invasive.

Selon un autre exemple de réalisation, la fibre optique comporte un revêtement tubulaire de protection d'une gaine optique contenant le coeur de la fibre optique, le coeur de la fibre et éventuellement la gaine optique étant enlevé sur une partie de l'extrémité de la fibre optique, afin de constituer la cavité de réception du matériau radioluminescent, cette cavité étant fermée par un matériau de protection.

Afin de réaliser une mesure différentielle, la sonde comporte une fibre optique supplémentaire qui n'est pas reliée au matériau luminescent et servant de référence, les fibres optiques étant connectées via le dispositif optique de sélection à deux tubes photomultiplicateurs identiques pour permettre de réaliser une mesure différentielle.

De préférence, chaque fibre optique est reliée à un connecteur qui est relié par une ou des fibres optiques de liaison au système de détection de luminescence.

Avantageusement, le système de détection de luminescence comporte au moins deux canaux de détection sur deux bandes spectrales différentes dont l'une est la bande étroite (BE) du matériau GaN.

Selon une caractéristique de l'invention, le système de détection de luminescence comporte en aval du dispositif optique de sélection, une unité de photodétection comportant un ou plusieurs tubes photomultiplicateurs.

Selon une variante de réalisation, le dispositif optique de sélection comporte un filtre optique passe bande centré sur le pic d'émission dans la bande étroite (BE) du matériau GaN.

Selon une autre variante de réalisation, le dispositif optique de sélection comporte un système optique dispersif ou diffractif permettant de séparer les composantes spectrales du signal avant d'être détectées sur deux canaux spectraux distincts à l'aide d'au moins deux tubes photomultiplicateurs, dont l'un sert à détecter la bande étroite (BE) et est pourvu de préférence d'une fente étroite.

Selon une autre variante de réalisation, le dispositif optique de sélection est réalisé par, de préférence, une lentille de collimation et par un système optique dispersif ou diffractif qui renvoie le signal vers l'unité de conversion photoélectrique réalisée à l'aide de tubes photomultiplicateurs multivoies.

Selon une application préférée, le système de détection de luminescence comporte des moyens de synchronisation de la fenêtre temporelle sur les impulsions de tirs à haute énergie relatives à un traitement de radiothérapie.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est un schéma de principe d'une sonde miniaturisée conforme à l'invention.
La **Figure 2A** présente le spectre d'émission de photoluminescence typique du GaN de type n pour différents niveaux de dopage silicium (intensité relative de photoluminescence I_{PL} en fonction de la longueur d'onde λ en nanomètre).
La **Figure 2B** présente le spectre de radioluminescence d'une fibre optique en silice fondue gainée en polymère (ETFB) (intensité de radioluminescence I_{RL} en fonction de la longueur d'onde λ en nanomètre).
Les **Figures 3A** à **3C** illustrent différents modes d'encapsulation du Nitrure de Gallium (GaN) dans une sonde miniaturisée conforme à l'invention.
La **Figure 4** illustre la réponse temporelle de radioluminescence aux impulsions de tirs d'irradiation provenant d'un accélérateur linéaire clinique.
Les **Figures 5A** à **5C** sont des schémas illustrant différents modes de réalisation du dispositif de mesure conforme à l'invention.

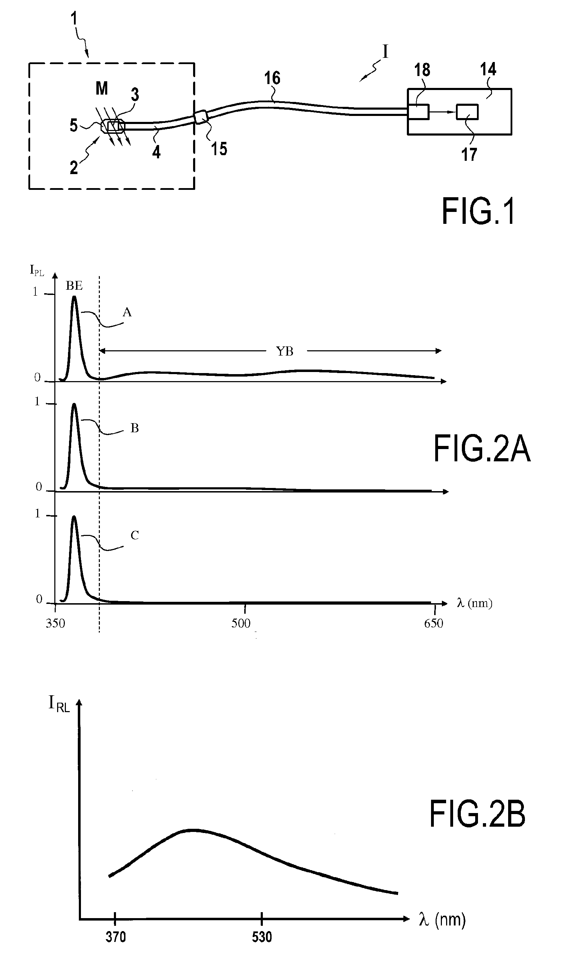

Tel que cela ressort plus précisément de la **Fig. 1**, l'objet de l'invention concerne une sonde miniaturisée **1** faisant partie d'un dispositif de dosimétrie **I** permettant de mesurer un rayonnement haute énergie **M** tel que les rayons X, γ, les électrons, les positrons et autres particules haute énergie. Cette sonde **1** comporte une cellule de conversion **2** comprenant un matériau radioluminescent **3** émettant un signal de luminescence dont l'intensité est fonction du rayonnement haute énergie **M** irradiant ledit matériau. Ce signal de luminescence est récupéré par au moins une fibre optique **4**.

Conformément à l'invention, le matériau radioluminescent **3** est du nitrure de gallium GaN, semi-conducteur III-V à gap direct (avec une énergie de gap Eg ∼ 3.4 eV à 300K) dont le numéro atomique (Z=19) est proche de celui des tissus biologiques. Ce matériau monocristallin intrinsèque ou non intentionnellement dopé possède typiquement un spectre de luminescence à température ambiante dans deux bandes spectrales distinctes, à savoir une bande étroite d'émission BE (Band Edge) et une bande large YB (Yellow Band).

Quand le matériau GaN est soumis à un rayonnement haute énergie, des paires d'électrons trous sont créés dans le matériau. La recombinaison radiative de ces porteurs à travers plusieurs canaux est prédominante et garantit un bon rendement de radioluminescence du GaN et un temps de réponse très court (de l'ordre de la nanoseconde). Le pic d'émission dans la bande étroite BE qui correspond au band gap du matériau est centré autour de 365 nm en longueur d'onde (**Fig. 2A**), tandis que l'émission large bande (YB) due aux défauts du matériau se produit à plus grande longueur d'onde et doit être minimisée pour les applications envisagées. L'émission de radioluminescence exclusivement dans la bande BE facilite la mise en oeuvre d'une discrimination spectrale du signal utile par rapport à l'émission large bande de la fibre optique **4** telle qu'elle apparaît sur la **Fig. 2B****.** De ce fait, la sonde **1** est avantageusement réalisée en utilisant un matériau GaN spécialement dopé pour que l'émission de radioluminescence se situe essentiellement dans la bande étroite BE du matériau GaN. Par exemple, le matériau GaN est de type n fortement dopé silicium (densité de dopants supérieure à 10¹⁹ atomes par cm³). En effet, comme cela apparaît sur la **Fig. 2A**, la répartition de l'émission de luminescence dans les deux bandes d'émission du GaN (BE et YB) dépend du niveau de dopage de ce matériau. Ainsi, à température ambiante, un matériau non intentionnellement dopé ou faiblement dopé silicium possède un spectre de luminescence dont la forme s'apparente à la courbe **A** alors que des matériaux moyennement (∼10¹⁸ atomes par cm³) et fortement (∼10¹⁹ atomes par cm³) dopés ont des allures de spectres respectivement proches des courbes **B** et **C**.

Selon une première variante de réalisation, le matériau GaN **3** est encapsulé dans la cellule de conversion **2** montée à l'une des extrémités de la fibre optique **4** pour former une sonde invasive particulièrement bien adaptée pour la dosimétrie in vivo en radiothérapie ou en radiodiagnostic. Par exemple, la cellule de conversion **2** est pourvue d'un revêtement **5** adapté pour assurer la fixation mécanique du matériau GaN **3** à l'extrémité de la fibre optique. Le choix du revêtement **5** prendra en compte les contraintes de l'application (par exemple la biocompatibilité pour la dosimétrie médicale) et l'optimisation de la collecte par la fibre optique du signal de luminescence. Par exemple, le revêtement **5** est du PTFE, de l'ETFE, du Polymide, du PEEK ou tout autre revêtement couramment utilisé pour les dispositifs médicaux invasifs

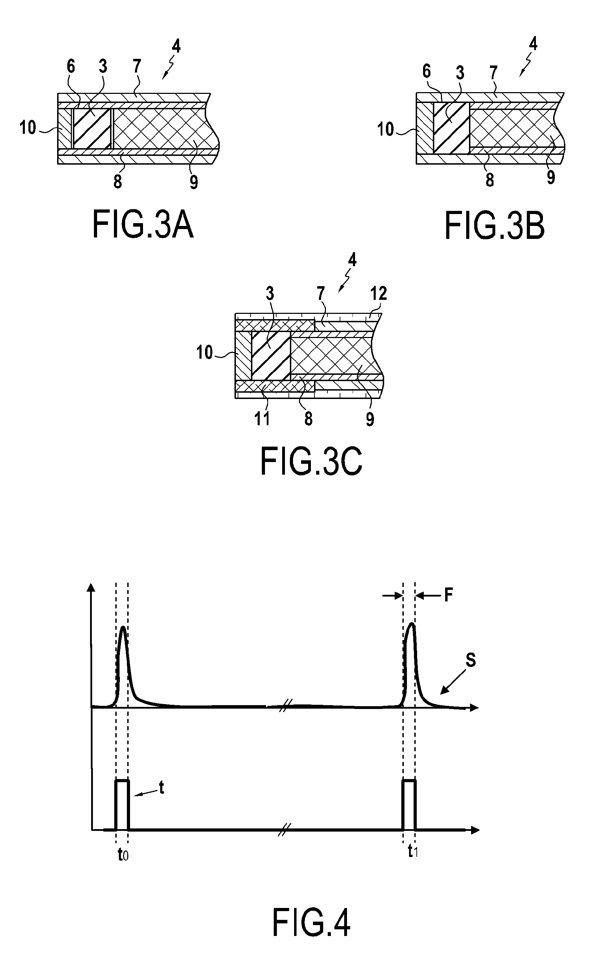

Les **Fig. 3A** à **3C** illustrent diverses autres variantes de montage du matériau GaN **3** à l'extrémité de la fibre optique **4.**

Dans les exemples illustrés aux **Fig. 3A** et **3B**, le matériau GaN **3** est logé dans une cavité **6** aménagée à l'extrémité de la fibre optique **4** qui comporte un revêtement tubulaire de protection **7** entourant une gaine optique **8** de la fibre optique **4**. Cette gaine optique **8** est au contact avec le coeur **9** de la fibre optique **4**. Dans l'exemple illustré à la **Fig. 3A**, la cavité **6** est aménagée directement dans le coeur **9** de la fibre optique **4** par une gravure humide ou sèche ou tout autre procédé adapté. Ainsi, le coeur **9** de la fibre optique **4** est enlevé sur une longueur déterminée à partir de son extrémité libre de la fibre, en laissant intacts la gaine optique **8** et le revêtement de protection **7** jusqu'à l'extrémité libre de la fibre optique **4**. Ainsi, toute une face du matériau GaN **3** est en contact avec le coeur **9** de la fibre optique **4**. Dans cette configuration, la gaine optique **8** entoure le matériau GaN **3** et de ce fait améliore le rendement de collecte du signal de luminescence, par la fibre optique **4**.

Dans l'exemple de réalisation illustré à la **Fig. 3B****,** le coeur **9** de la fibre **4** et la gaine optique **8** sont enlevés sur une longueur déterminée à partir de l'extrémité libre de la fibre, en laissant intact le revêtement de protection **7** jusqu'à l'extrémité libre de la fibre. Selon cet exemple, le matériau GaN **3** est en contact avec l'extrémité du coeur **9** et de la gaine optique **8.**

Dans les exemples décrits aux **Fig. 3A** et **3B**, il est à noter que la cavité **6** possède une longueur (prise dans l'axe de la fibre optique **4**) supérieure à la longueur du matériau GaN **3** pour permettre l'insertion d'un matériau de protection **10** venant fermer l'extrémité de la fibre optique **4.** Ce matériau de protection **10** du type biocompatible vient emprisonner le matériau GaN **3** dans la cavité **6** en étant inséré dans la section d'extrémité de la gaine optique **8** (**Fig**. **3A**) ou du revêtement de protection **7 (****Fig. 3B****)**.

Ces variantes de réalisation permettent le montage étanche du matériau GaN **3** dans le prolongement de la fibre optique sans augmenter le diamètre externe de la fibre optique **4.** Par exemple, pour la dosimétrie in vivo, le diamètre externe de la sonde peut être inférieur à 800 microns.

La **Fig. 3C** illustre un autre exemple de réalisation pour lequel la cavité de réception **6** du matériau GaN **3** est réalisée par un tube **11** manchonné à l'extrémité de la fibre optique, sur la gaine optique **8**, et fermée par un matériau de protection **10**. Le tube **11** et le revêtement **7** peuvent être insérés à l'intérieur d'une enveloppe de protection **12**.

L'interface entre le matériau GaN **3** et l'extrémité de la fibre optique **4** peut inclure des moyens adéquats pour optimiser le rendement de couplage optique, tels que par exemple des gels d'adaptation d indice, lentille à gradient d'indice (GRIN).

La fibre optique **4** reçoit le signal radioluminescent émis par le matériau GaN **3** et assure sa transmission vers un système de détection de luminescence **14**.

Selon une caractéristique de réalisation, la fibre optique **4** est reliée via un connecteur **15**, à une fibre optique de liaison **16** connectée au système de détection de luminescence **14**. La sonde **1** munie de la fibre optique **4** forme ainsi un dispositif à usage unique jetable. La fibre optique de liaison **16** qui peut atteindre quelques dizaines de mètres permet d'éloigner le système de luminescence **14** par rapport à la sonde de mesure **1**, et donc par rapport à la zone d'irradiation.

Le système de détection de luminescence **14** comporte des moyens de synchronisation de la fenêtre temporelle **F** sur les impulsions de tirs relatives au traitement de radiothérapie. Ainsi, tel que cela ressort de la **Fig. 4**, le signal de luminescence **S** est pris en compte dès le début du tir haute énergie **t** irradiant la zone de traitement concernée. Typiquement, chaque tir haute énergie **t** possède une durée de 5 microsecondes. Chaque fenêtre temporelle **F** permet de prendre en compte le signal de radioluminescence **S** à chaque début **tᵢ** des tirs **t** et pendant toute la durée de chaque tir **t**. Ce fenêtrage temporel permet d'améliorer significativement le rapport signal à bruit de détection de la radioluminescence et la précision de mesure. Par exemple, l'analyse temporelle du signal de radioluminescence associée à la rapidité du système de détection peut permettre de discriminer les différentes contributions de luminescence sur la base de leurs propriétés temporelles, et de s'affranchir de scintillations parasites lentes.

De manière classique, le système de détection de luminescence **14** comporte une unité de photodétection **17** réalisant la conversion photoélectrique du signal de luminescence permettant de mesurer le rayonnement haute énergie irradiant le matériau GaN **3**. Selon une caractéristique de l'invention, l'unité de photodétection **17** comporte un ou plusieurs tubes photomultiplicateurs mono ou multivoies (multianodes). L'avantage de recourir à un tube photomultiplicateur en tant que photodétecteur est d'obtenir une haute sensibilité de détection et des temps de réponse courts afin d'avoir une bonne résolution temporelle sur le signal détecté.

Selon une autre caractéristique de l'objet de l'invention, le système de détection de luminescence **14** comporte un dispositif optique **18** permettant de sélectionner la bande étroite BE d'émission du matériau GaN **3.** Ce dispositif optique **18** est réalisé par tout moyen optique approprié et il est monté en amont de l'unité de photodétection **17**.

Selon un exemple de réalisation, le dispositif optique de sélection **18** est réalisé par un filtre optique passe bande recevant le signal de luminescence transmis par la fibre optique de liaison **16**. Le filtre optique passe bande est centré sur le pic d'émission dans la bande BE du matériau GaN **3**, c'est-à-dire autour de 365 nm. Le signal ainsi filtré est détecté par l'unité de photodétection **17** réalisée par un tube photomultiplicateur.

Cette mise en oeuvre s'applique avantageusement lorsque les contributions parasites sont négligeables dans la bande d'émission BE du matériau GaN par exemple pour des irradiations très localisées (faible champ). Dans cette configuration, le volume de fibre optique irradié est suffisamment faible pour que l'émission de radioluminescence associée soit négligeable devant celle du matériau GaN dans la bande passante du filtre.

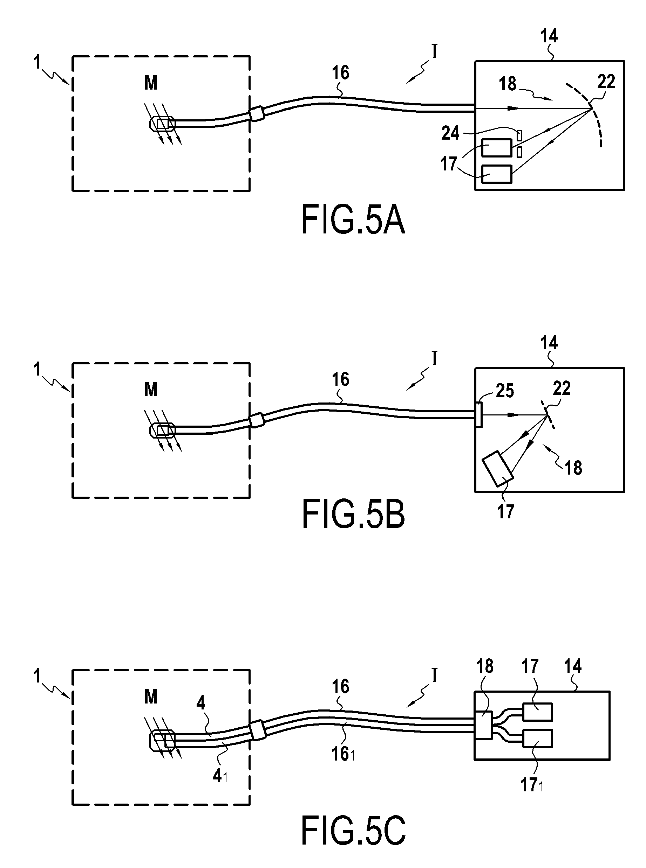

La **Fig. 5A** illustre un autre exemple de réalisation du système de détection de luminescence **14** transmis par la fibre optique de liaison **16**. Le signal de luminescence est dirigé vers le dispositif optique de sélection **18** réalisé par un système optique dispersif ou diffractif **22** (réseau de diffraction, prisme, etc) ou, comme illustré, un réseau concave en réflexion. Les composantes spectrales du signal ainsi séparées spatialement par un tel système optique **22** sont détectées sur deux canaux spectraux distincts à l'aide d'au moins deux tubes photomultiplicateurs **17**. Avantageusement, devant le tube photomultiplicateur **17** qui sert à détecter la composante utile (Bande BE du GaN), il est possible d'ajouter une fente étroite **24** permettant d'augmenter la sélectivité spectrale. Cette variante permet de mesurer d'une part la luminescence du matériau GaN dans la bande BE et d'autre part d'estimer les contributions des luminescences parasites dans une bande spectrale distincte.

La **Fig. 5B** illustre une autre variante de réalisation d'un système de détection de luminescence **14**. Le signal de luminescence transmis par la fibre optique **16** est dirigé vers le dispositif optique de sélection **18** réalisé par, de préférence, une lentille de collimation **25** et par un système optique dispersif ou diffractif **22** qui renvoie le signal vers l'unité de photodétection **17** réalisée à l'aide de tubes photomultiplicateurs multivoies (multianodes). Cette configuration permet d'obtenir le spectre du signal de luminescence provenant de la sonde. A partir de ces informations spectrales, il est possible d'estimer l'intensité de luminescence du matériau GaN en retranchant les contributions parasites. Cette analyse spectrale permet une meilleure estimation des différentes contributions des signaux utiles et parasites par rapport à une méthode sur deux canaux comme celle décrite sur la figure 5A.

Il est à noter que les variantes de réalisation décrites jusqu'ici n'utilisent qu'un seul canal à fibre optique et qu'elles permettent de rejeter la contribution des luminescences parasites en exploitant des informations dans le domaine spectral. Cette approche a l'avantage d'être bien adaptée pour réaliser une sonde miniaturisée.

La **Fig. 5C** illustre une autre variante de réalisation permettant d'effectuer une mesure différentielle. Selon cette variante, le dispositif comporte une fibre optique de référence **4₁** et une fibre optique de liaison de référence **16₁** qui sont identiques respectivement à la fibre optique **4** et à la fibre optique de liaison **16**. Cette fibre optique de référence **4₁** n'est reliée à aucun matériau radioluminescent tandis que la fibre optique de liaison de référence **16₁** est connectée à un tube photomultiplicateur **17₁** identique au tube photomultiplicateur **17**. Bien entendu, en amont des tubes photomultiplicateurs **17**, **17₁,** il est interposé l'une ou l'autre des variantes de réalisation du dispositif optique de sélection **18.**

Un des avantages de la variante décrite à la **Fig. 5C** est de permettre de retrancher les contributions parasites du signal utile pour améliorer la précision de mesure et la sensibilité.

Il doit être considéré que la sonde **1** conforme à l'invention peut être miniaturisée dans la mesure où elle est réalisée essentiellement par une fibre optique à l'extrémité de laquelle est placée la cellule de détection comportant essentiellement le matériau GaN conforme à l'invention. Cette sonde qui présente un faible coût de revient peut posséder un caractère jetable. Cette sonde est particulièrement adaptée pour constituer une sonde invasive à usage unique. Un autre avantage d'un tel dispositif concerne la possibilité de mesurer en temps réel la dose de rayonnement haute énergie. Un tel dispositif permet de s'affranchir des connexions électriques entre la cellule de détection **2** et le tube photomultiplicateur **17**.

## Revendications

1. Dispositif pour la dosimétrie in vivo, comportant :
• une sonde miniaturisée (**1**) comprenant au moins :
- un matériau radioluminescent (**3**) émettant un signal de luminescence dont l'intensité est fonction du rayonnement haute énergie irradiant ledit matériau,
- et une fibre optique (**4**, **16**) recevant le signal de luminescence et assurant sa transmission vers un système de détection de luminescence (**14**),
• et un système de détection de luminescence (**14**),
**caractérisé en ce que** le matériau radioluminescent (**3**) est du nitrure de gallium GaN spécifiquement dopé afin d'émettre un signal de luminescence essentiellement dans la bande étroite d'émission (**BE**) du matériau qui correspond au band gap du matériau, et **en ce que** le système de détection de luminescence (**14**) comporte un dispositif optique (**18**) permettant de sélectionner la bande étroite BE d'émission du nitrure de gallium.

2. Dispositif selon la revendication 1, **caractérisé en ce que** le matériau radioluminescent GaN est placé dans une cavité de détection (**6**) montée à l'extrémité d'une fibre optique ou réalisée à l'une des extrémités d'une fibre optique pour former une sonde invasive.

3. Dispositif selon la revendication 2, **caractérisé en ce que** la fibre optique (**4**) comporte un revêtement tubulaire (**7**) de protection d'une gaine optique (**8**) contenant le coeur (**9**) de la fibre optique, le coeur (**9**) de la fibre et éventuellement la gaine optique (**8**) étant enlevé sur une partie de l'extrémité de la fibre optique, afin de constituer la cavité de réception (**6**) du matériau radioluminescent (**3**), cette cavité étant fermée par un matériau de protection (**10**).

4. Dispositif selon l'une des revendications 1 à 3, **caractérisé en ce que** la sonde (**1**) comporte une fibre optique de référence (**4₁**) identique à celle (**4**) reliée au matériau luminescent (**3**), mais non reliée à un matériau radioluminescent, les fibres optiques (**4**) et de référence (**4₁**) étant connectées via le dispositif optique de sélection (**18**) à deux tubes photomultiplicateurs identiques (**17, 17₁**) pour permettre de réaliser une mesure différentielle.

5. Dispositif selon l'une des revendications 1 à 4, **caractérisé en ce que** chaque fibre optique (**4, 4₁**) est reliée à un connecteur (**15**) qui est relié par une ou des fibres optiques de liaison (**16, 16₁**) au système de détection de luminescence (**14**).

6. Dispositif selon l'une des revendications 1 à 5, **caractérisé en ce que** le système de détection de luminescence (**14**) comporte au moins deux canaux de détection sur deux bandes spectrales différentes dont l'une est la bande étroite (BE) du matériau GaN.

7. Dispositif selon l'une des revendications 1 à 6, **caractérisé en ce que** le système de détection de luminescence (**14**) comporte en aval du dispositif optique de sélection (**18**), une unité de photodétection (**17**) comportant un ou plusieurs tubes photomultiplicateurs.

8. Dispositif selon l'une des revendications 1 à 7, **caractérisé en ce que** le dispositif optique de sélection (**18**) comporte un filtre optique passe bande centré sur le pic d'émission dans la bande étroite (BE) du matériau GaN (**3**).

9. Dispositif selon l'une des revendications 1 à 7, **caractérisé en ce que** le dispositif optique de sélection (**18**) comporte un système optique dispersif ou diffractif (**22**) permettant de séparer les composantes spectrales du signal avant d'être détectées sur deux canaux spectraux distincts à l'aide d'au moins deux tubes photomultiplicateurs (**17**), dont l'un sert à détecter la bande étroite (BE) et est pourvu de préférence d'une fente étroite (**24**).

10. Dispositif selon l'une des revendications 1 à 7, **caractérisé en ce que** le dispositif optique de sélection (**18**) est réalisé par, de préférence, une lentille de collimation (**25**) et par un système optique dispersif ou diffractif (**22**) qui renvoie le signal vers l'unité de conversion photoélectrique (**17**) réalisée à l'aide de tubes photomultiplicateurs multivoies.

11. Dispositif selon l'une des revendications 1 à 10, **caractérisé en ce que** système de détection de luminescence (**14**) comporte des moyens de synchronisation de la fenêtre temporelle (**F**) sur les impulsions de tirs (**t**) à haute énergie relatives à un traitement de radiothérapie.

## Patentansprüche

1. In-Vivo-Dosimetrievorrichtung, umfassend:
- eine Miniatursonde (1), mindestens umfassend:
- ein radiolumineszierendes Material (3), das ein Lumineszenzsignal entsendet, dessen Stärke von der hochenergetischen Strahlung, die das Material bestrahlt, abhängt,
- und eine optische Faser (4, 16) die das Lumineszenzsignal empfängt und seine Übertragung zu einem Lumineszenznachweissystem (14) gewährleistet,
- und ein Lumineszenznachweissystem (14),
**dadurch gekennzeichnet, dass** das radiolumineszierende Material (3) Galliumnitrid GaN ist, das spezifisch dotiert ist, um ein Lumineszenzsignal im Wesentlichen in dem schmalen Sendeband (BE) des Materials zu entsenden, das dem Bandabstand des Materials entspricht, und dass das Lumineszenznachweissystem (14) eine optische Vorrichtung (18) umfasst, die es ermöglicht, das schmale Sendeband (BE) des Galliumnitrids auszuwählen.

2. Vorrichtung nach Anspruch 1, **dadurch gekennzeichnet, dass** das radiolumineszierende Material GaN in einem Nachweishohlraum (6) angeordnet ist, der am Ende einer optischen Faser montiert oder an einem der Enden einer optischen Faser hergestellt ist, um eine invasive Sonde zu bilden.

3. Vorrichtung nach Anspruch 2, **dadurch gekennzeichnet, dass** die optische Faser (4) eine röhrenförmige Schutzverkleidung (7) einer optischen Hülle (8) umfasst, die den Kern (9) der optischen Faser enthält, wobei der Kern (9) der Faser und eventuell die optische Hülle (8) auf einem Teil des Endes der optischen Faser entfernt sind, um den Aufnahmehohlraum (6) des radiolumineszierenden Materials (3) zu bilden, wobei dieser Hohlraum durch ein Schutzmaterial (10) geschlossen ist.

4. Vorrichtung nach einem der Ansprüche 1 bis 3, **dadurch gekennzeichnet, dass** die Sonde (1) eine optische Referenzfaser (4₁) identisch mit jener (4), die mit dem lumineszierenden Material (3) verbunden, aber nicht mit einem radiolumineszierenden Material verbunden ist, umfasst, wobei die optische Faser (4) und Referenzfaser (4₁) über die optische Auswahlvorrichtung (18) an zwei identische Fotovervielfachungsröhren (17, 17₁) angeschlossen sind, um die Durchführung einer Differentialmessung zu ermöglichen.

5. Vorrichtung nach einem der Ansprüche 1 bis 4, **dadurch gekennzeichnet, dass** jede optische Faser (4, 4₁) mit einem Stecker (15) verbunden ist, der über eine oder mehrere optische Verbindungsfasern (16, 16₁) mit dem Lumineszenznachweissystem (14) verbunden ist.

6. Vorrichtung nach einem der Ansprüche 1 bis 5, **dadurch gekennzeichnet, dass** das Lumineszenznachweissystem (14) mindestens zwei Nachweiskanäle auf zwei unterschiedlichen Spektralbändern umfasst, von denen eines das schmale Band (BE) des Materials GaN ist.

7. Vorrichtung nach einem der Ansprüche 1 bis 6, **dadurch gekennzeichnet, dass** das Lumineszenznachweissystem (14) stromabwärts zu der optischen Auswahlvorrichtung (18) eine Fotodetektionseinheit (17) umfasst, die eine oder mehrere Fotovervielfachungsröhren umfasst.

8. Vorrichtung nach einem der Ansprüche 1 bis 7, **dadurch gekennzeichnet, dass** die optische Auswahlvorrichtung (18) einen optischen Bandpassfilter umfasst, der auf die Emissionsspitze in dem schmalen Band (BE) des Materials GaN (3) zentriert ist.

9. Vorrichtung nach einem der Ansprüche 1 bis 7, **dadurch gekennzeichnet, dass** die optische Auswahlvorrichtung (18) ein dispersives oder diffraktives optisches System (22) umfasst, das es ermöglicht, die Spektralkomponenten des Signals zu trennen, bevor sie auf zwei verschiedenen Spektralkanälen mit Hilfe mindestens einer der Fotovervielfachungsröhren (17) erfasst werden, von denen eine dazu dient, das schmale Band (BE) zu erfassen, und vorzugsweise mit einem schmalen Schlitz (24) versehen ist.

10. Vorrichtung nach einem der Ansprüche 1 bis 7, **dadurch gekennzeichnet, dass** die optische Auswahlvorrichtung (18) vorzugsweise durch eine Kollimationslinse (25) und ein dispersives oder diffraktives optisches System (22) verwirklicht ist, das das Signal zu der fotoelektrischen Konversionseinheit (17), die mit Hilfe von Mehrwege-Fotovervielfachungsröhren verwirklicht ist.

11. Vorrichtung nach einem der Ansprüche 1 bis 10, **dadurch gekennzeichnet, dass** das Lumineszenznachweissystem (14) Mittel zur Synchronisation des Zeitfensters (F) auf die Impulse von Hochenergiebeschüssen (t) im Zusammenhang mit einer Radiotherapiebehandlung umfasst.

## Claims

1. A device for in vivo dosimetry, the device comprising:
• a miniature probe (1) comprising at least:
• a radioluminescent material (3) that emits a radioluminescence signal of intensity that is a function of the high-energy radiation irradiating said material; and
• an optical fiber (4, 16) receiving the luminescence signal and conveying it to a luminescence detector system (14); and
• a luminescence detector system (14);
**characterized in that** the radioluminescent material (3) is gallium nitride (GaN) specifically doped so that to emit a luminescence signal essentially in the narrow emission band (BE) of the material that corresponds to the band gap of the material, and **in that** the luminescence detector system (14) includes an optical device (18) enabling the narrow emission band BE of gallium nitride to be selected.

2. A device according to claim 1, **characterized in that** the GaN radioluminescent material is placed in a detection cavity (6) mounted on the end of an optical fiber or made at one of the ends of an optical fiber in order to form an invasive probe.

3. A device according to claim 2, **characterized in that** the optical fiber (4) includes a tubular covering (7) for protecting optical cladding (8) that contains the core (9) of the optical fiber, the core (9) of the fiber and optionally the cladding (8) being removed over an end portion of the optical fiber in order to constitute the cavity (6) for receiving the radioluminescent material (3), this cavity being closed by a protective material (10).

4. A device according to any one of claims 1 to 3, **characterized in that** the probe (1) includes a reference optical fiber (4₁) identical to the fiber (4) connected to the luminescent material (3), but not connected to any radioluminescent material, the optical fiber (4) and the reference optical fiber (4₁) being connected via the optical selector device (18) to two identical photomultiplier tubes (17, 17₁) to enable differential measurements to be performed.

5. A device according to any one of claims 1 to 4, **characterized in that** each optical fiber (4, 4₁) is connected to a connector (15) that is connected via one or more link optical fibers (16, 16₁) to the luminescence detector system (14).

6. A device according to any one of claims 1 to 5, **characterized in that** the luminescence detector system (14) includes at least two detection channels on two different spectrum bands, one of which is the narrow band (BE) of GaN material.

7. A device according to any one of claims 1 to 6, **characterized in that** the luminescence detector system (14) includes, downstream from the optical selector device (18), a photodetector unit (17) comprising one or more photomultiplier tubes.

8. A device according to any one of claims 1 to 7, **characterized in that** the optical selector device (18) comprises a bandpass optical filter centered on the emission peak in the narrow band (BE) of GaN material (3).

9. A device according to any one of claims 1 to 7, **characterized in that** the optical selector device (18) comprises a dispersive or diffractive optical system (22) enabling the spectral components of the signal to be separated prior to being detected on two distinct spectrum channels using at least two photomultiplier tubes (17), one of which serves to detect the narrow band (BE) and is preferably provided with a narrow slit (24).

10. A device according to any one of claims 1 to 7, **characterized in that** the optical selector device (18) is preferably constituted by a collimator lens (25) and by a dispersive or diffractive optical system (22) that delivers the signal to the photoelectrical converter unit (17) made with the help of multichannel photomultiplier tubes.

11. A device according to any one of claims 1 to 10, **characterized in that** the luminescence detector system (14) includes means for synchronizing the time window (F) on the high-energy pulse shots (t) relating to radio therapy treatment.
